Europäisches Patentamt

European Patent Office

Office européen des brevets

(19)

(11) Veröffentlichungsnummer: **0 278 035**
**B1**

(12) **EUROPÄISCHE PATENTSCHRIFT**

(45) Veröffentlichungstag der Patentschrift: 23.01.91

(21) Anmeldenummer: **87101996.4**

(22) Anmeldetag: **12.02.87**

(51) Int. Cl.⁵: **C 12 P 1/04, C 12 N 1/20,**
**A 01 N 63/00** // (C12P1/04,
C12R1:07),(C12N1/20,
C12R1:07)

(54) Sporenlose Mutanten des Bacillus thuringiensis serovar. israelensis und Toxine daraus.

(43) Veröffentlichungstag der Anmeldung:
17.08.88 Patentblatt 88/33

(45) Bekanntmachung des Hinweises auf
die Patenterteilung:
23.01.91 Patentblatt 91/04

(84) Benannte Vertragsstaaten:
AT BE CH DE FR GB IT LI NL SE

(56) Entgegenhaltungen:
EP-A-0 059 460
EP-A-0 099 301

APPLIED AND ENVIRONMENTAL
MICROBIOLOGY, Band 43, Nr. 6, Juni 1982,
Seiten 1473-1480; Y. WAKISAKA et al.:
"Formation of Crystalline delta-endotoxin or
poly-beta-hydroxybutyric acid granules by
asporogenous mutants of Bacillus
thuringiensis"

(73) Patentinhaber: BASF Aktiengesellschaft
Carl-Bosch-Strasse 38
D-6700 Ludwigshafen (DE)

(72) Erfinder: Zaehner, Hans, Prof. Dr.
Im Hopfengarten 13
D-7400 Tuebingen (DE)
Erfinder: Bernhard, Konrad, Dr.
Jessinger Hauptstrasse 132
D-7400 Tuebingen (DE)
Erfinder: Weisser, Harald, Dr.
Buchenstrasse 2 (Reindl)
D-8122 Penzberg (DE)

Courier Press, Leamington Spa, England.

# EP 0 278 035 B1

**Beschreibung**

Neben den synthetischen und den aus höheren Pflanzen gewonnenen Insektiziden sind bakterielle Insektizide bekannt: verschiedene Typen des seit 1915 bekannten Bacillus thuringiensis sind die Wirkstoffproduzenten:

Die Wildform des insektenpathogens Bakterium B.thuringiensis ist fakultativ anaerob und in der Lage Endosporen zu bilden. Sie zeichnet sich gegenüber anderen sporenbildenden Bakterien dadurch aus, daß während der Sporulation im Sporangium ein parasporaler Proteinkristall gebildet wird. Das Kristallprotein wirkt als Fraßgift auf Insekten und wird als delta-Endotoxin bezeichnet. Die Pathogenität von B.thuringiensis beruht im wesentlichen auf der Toxizität des Kristallproteins; Varianten von B.thuringiensis, welche nicht mehr in der Lage sind Kristalle zu bilden, sind auch nicht mehr pathogen für Insekten.

Die parasporalen Kristalle bestehen aus Glycoproteinen mit Molekulargewichten von etwa 120,000. Die Proteinmoleküle sind im Kristall durch Disulfidbrücken kovalent miteinander verbunden. Die Kristalle sind bei neutralem pH unlöslich in wäßrigen und organischen Lösungsmitteln. Im alkalischen pH-Bereich, oberhalb pH 9.0 lösen sie sich auf, wobei das Kristallprotein in kleinere lösliche Peptide mit Molekulargewichten zwichen ca. 23,000 und 70,000 gespalten wird. Unter den so entstandenen Peptiden befindet sich auch das aktive Toxin.

Die delta-Endotoxine unterscheiden sich von anderen für Insketen toxischen Substanzen durch die hohe Toxizität gegen sensitive Insektenlarven und die ausgeprägte Spezifität d.h. das Fehlen von Toxizität gegen andere Lebewesen, insbesondere Säugern. Ihre chemische Struktur läßt erwarten, daß sie ohne Rückstände biologisch abgebaut werden können.

Diese Eigenschaften legen es nahe, die delta-Endotoxine als Insektizide im Pflanzenschutz einzusetzen. In der Tat werden seit einigen Jahrzehnten Präparate auf der Basis von B.thuringiensis angeboten. Für ihre Herstellung werden Stämme verwendet, die dem Pathotyp A angehören. Sie werden vor allem im Obstbau gegen bestimmte Arten von Schmetterlingslarven verwendet.

Im Jahr 1977 wurden — später mit "A 60" oder Bacillus thuringiensis serovar. israelensis (BTI) bezeichnete — Isolate von B.thuringiensis beschrieben, deren delta-Endotoxine für die Larven von Dipteren toxisch sind.

Eine andere, ebenfalls in der Literatur verwendete Bezeichnung für den Bacillus ist B. cereus israelensis.

Seit etlichen Jahren sind Versuche im Gange auf der Basis solcher Stämme Präparate herzustellen, die zur Bekämpfung von Stech- und Kriebelmücken geeignet sind (Pathotyp B).

Bei der Verwendung von Präparaten auf der Basis von Stämmen des Pathotyps B. von B.thuringiensis treten allerdings Probleme auf: Die Brutplätze von Mücken sind Oberflächengewässer, in welche Präparate auf der Basis von B.thuringiensis serovar. israelensis eingebracht werden müssen. Aus Gründen des Gewässerschutzes ist das Ausbringen von keimfähigen Sporen in Gewässer untunlich, da es die Gefahr der unkontrollierten Ausbreitung von gewässerfremden Mikroorganismen birgt. Da jede sporulierende Zelle je eine Spore und einen Proteinkristall produziert, sind in bisher erhältlichen Präparaten Proteinkristalle und keimfähige Sporen in gleicher Anzahl erhalten. Im Versuchmaßstab ist est ohne weiteres möglich. Sporen und Kristalle voneinander zu trennen. Die dafür verwendeten Verfahren, wie z.B. Dichtegradientenzentrifugation oder Zweiphasentrennung sind aber im technischen Maßstab nicht praktikabel. Durch Bestrahlung mit UV- oder Gamma-Strahlen können die Sporen abgetötet werden, ohne daß dadurch die insektizide Aktivität des Kristallproteins herabgesetzt wird. Die Sterilisierung von B.thuringiensis-Präpareten durch UV- oder Gamma-Bestrahlung ist jedoch teuer und deshalb wirtschaftlich nicht zu vertreten.

Ein weiteres Problem besteht darin, daß die spezifische Aktivität der so erhaltenen Präparate noch zu niedrig ist. Auch hier ist eine der Ursachen, daß die Präparate neben den Proteinkristallen noch inaktive Sporen und z.T. auch vegetative Zellen enthalten. Dadurch wird der eigentliche Wirkstoff mehr oder minder stark verdünnt. Durch die Sterilization des Rohpräparates mit Gammastrahlen werden Sporen und vegetative Zellen zwar abgetötet, bleiben aber noch als Partikel im Präparat. Zwar könnte bei manchen Anwendungs- bzw. Ausbringungsformen der Toxine eine gewisse Verdünnung in Kauf genommen werden. Die praktische Zubereitung der Toxine (die sog. Formulierung) führt jedoch ohnehin zwangsweise zur Verdünnung, z.B. weil Beschwerungsstoffe zugesetzt oder die Präparate thermisch (etwa bei oder Sprühtrocknung) belastet werden müssen.

Die beschriebenen Schwierigkeiten würden vermieden, wenn es gelänge, ein Verfahren zur Fermentation von mückenpathogenen Stämmen von B.thuringiensis zu entwickeln, bei dem von vornherein nur Proteinkristalle und keine Sporen und vegetativen Zellen entstehen und somit ein hochkonzentriertes Präparat erhalten wird.

In der europäischen Patentveröffentlichung 99 301 ist u.a. eine sporenlose Mutante von dipterentoxischem Bacillus thuringiensis israelensis (bzw. B. cereus ssp. israelensis, BCI) beschrieben mit der Stammbezeichnung CB 3—104R bzw. der Hinterlegungsnummer 262 des Canadian Committee on Culture Collections bzw. ATCC 39, 152 der American Type Culture Collection.

Diese Mutante ist zwar in der Lage, sporenfreie Zubereitungen des Kristallienen Toxins zu liefern, jedoch nur, wenn alle Individuen derselben Kultur sich stets im gleichen Stadium der Sporulation befinden,

2

da es sich bei dieser Mutante nicht um eine sporenose Mutante im eigentlichen Sinne handelt, sondern um einen Organismus, der die Endospore nach der Bildung des parasporalen Kristalls wieder auflöst. Die praktische Unmöglichkeit, im technischen Maßstab Kulturen zu ziehen, die Organismen einheitlichen Alters enthält, hat zur Folge, daß es mit der Mutante der EP-Veröffentlichung 99 301 nicht gelingt, sporen- und zellfreie Präparate des toxins unmittelbar, d.h. ohne weitergehende Maßnahmen zu gewinnen.

Die EP—A—99 301 enthält jedoch zur Phänomenologie und Bearbeitungspraxis von BTI zahlreiche Hinweise, weshalb sie zur Ergänzung der nachfolgenden Beschreibung herangezogen werden kann, wo dies erforderlich erscheint.

Eine ebenfalls mit der Züchtung sporenloser Mutanten von B.thuringiensis (serovar, kurstaki) befaßte Veröffentlichung ist die EP—A—59 460 auf die ergänzend hinzuweisen ist, ebenso wie auf die Vorschläge, die genetische Information, die zur Erzeugung des Toxins führt, in einen Wirtsorganismus zu übertragen (z.B. E. coli), die etwa in der EP—A—63 949 enthalten sind.

Es wurde nun eine sporenlose Mutante des Bacillus thuringiensis serovar, israelensis gefunden, welche unter der Nummer DSM 3440 bei der Deutschen Sammlung von Mikroorganismen hinterlegt wurde. Diese nachstehend als HA-9 bezeichnete Mutante liefert unter gewissen Umständen sporen- und zellmaterial-freie Toxinpräparate, ohne daß es dazu eines besonderen Sterilisationsschrittes bedarf.

Weiterhin wurde eine sporenlose Mutante des Bacillus thuringiensis serovar. israelensis gefunden, welche unter der Nummer DSM 3439 bei der Deutschen Sammlung von Mikroorganismen hinterlegt wurde. Diese Mutante wird im folgenden mit HA-5 bezeichnet.

Weiterhin wurde ein Verfahren zur Herstellung ballastarmer und sporenfreier mückentoxischer Präparate aus Bacillus thuringiensis servor.israelensis gefunden, das dadurch gekennzeichnet ist, daß man, gegebenenfalls nach Einwirkung von die Mutationsrate erhöhenden Mitteln aus wachsenden Bakterienkolonien diejenigen ausliest, die bei einer Fermentationstemperatur von unter 30°C thermisch instabile Sporen bilden, deren vegetative Zellen vermehrt und aus dem Substrat in an sich bekannter Weise das Toxin gewinnt.

Darüber hinaus wurde ein Verfahren zur Herstellung konzentrierter, ballastarmer und sporenfreier mückentoxischer Proteinpräparate aus Bacillus thuringiensis serovar.israelensis gefunden, das dadurch gekennzeichnet ist, daß man, gegebenenfalls nach Einwirkung von die Mutationsrate erhöhenden Mitteln, aus wachsenden Bakterienkolonien diejenigen ausliest, die ausschließlich Präsporen bilden, diese in einem geeigneten Kulturmedium bei einer Temperatur von unter 30°C vermehrt, bis die Sporulation eingetreten ist und danach bei einer Temperatur von über 35°C hält, die zur Autolyse der Sporen führt, sowie das Toxin aus dem Kulturmedium gewinnt.

Gegenstand der Erfindung sind ferner die Verwendung der nach den oben genannten Verfahren erhältlichen Toxine zur Bekämpfung von Stechmücken.

Erfindungsgemäß wird ferner ein Insektizid zur Verfügung gestellt, das dadurch gekennzeichnet ist, daß es als aktiven Wirkstoff Bacillus thuringiensis serovar.israelensis DSM 3439 oder ein hieraus erhältliches Toxin enthält, sowie ein Insektizid, das dadurch gekennzeichnet ist, daß es als aktiven Wirkstoff Bacillus thuringiensis serovar.israelensis DSM 3440 oder ein hieraus erhältliches Toxin enthält.

Beschreibung des Ausgangsstammes

Im Jahre 1977 wurden von Goldberg und Margalit an einer Brutstelle von Stechmücken in der Negev-Wüste in Israel ein Bacillus mit insektizider Wirkung gegen Larven von Stechmücken isoliert. Dieses mit A-60 bezeichnete Isolat wurde von deBarjac als B.thuringiensis identifiziert (C.R. Acad. Sci., Paris, *286*, 797—800 und 1175—1178, Serie D, (1978)). Da es dem bis dahin unbekannten Pathotyp B und dem damals ebenfalls neuen Flagellenserotyp H-14 angehört, wird es als B.thuringiensis serovar.israelensis bezeichnet. Dieser Stamm bildet das Ausgangsmaterial für den erfindungsgemäßen Mikroorganismus.

In den letzten Jahren zind noch weitere Isolate beschrieben worden, die ebenfalls dem Pathotyp B, aber den Flagellenserotypen H-8, H-10 und H-11 angehören. Die spezifische Toxizität der Kritalltoxine aus diesen Isolaten ist jedoch niedriger als bei Stamm A-60.

Deshalb wurde bei den nachstehend beschriebenen bzw. den erfindungsgemäß beschriebenen Mutanten immer von Stamm A-60 ausgegangen.

Beschreibung der Sporulation in B.thuringiensis

Die Produktion von Endosporen bei Bacillen setzt in der Regel in der stationären Wachstumsphase ein. Sie ist als Reaktion auf die Erschöpfung der für die Zelle verwertbaren Substrate, und die damit verbundenen Verschlechterungen der Lebensbedingungen anzusehen. Vegetative Zellen sind biochmisch aktiv, teilungsfähig und sensitiv gegen Hitze und Austrocknung. Endosporen besitzen dagegen eine beträchtliche Resistenz gegen Hitze und Austrocknung, sind aber biochemisch inaktiv und nicht teilungsfähig. Sie stellen ein Ruhestadium dar. das Jahrzehnte dauern kann. Es wird beendet, wenn die Spore mit Substraten in Kontakt kommt, die eine Vermehrung ermöglichen, z.B. eine Nährlösung im Laboratorium. In einem Prozeß, der als Keimung bezeichnet wird, wandelt sich dabei die Spore in eine vegetative Zelle um. Bei dem Vorgang der Sporenbildung, im weiteren Text als Sporulation bezeichnet, finden in der sporulierenden Zelle beträchtliche morphologische Änderungen statt. Zur besseren Beschreibung wird die Sporulation in 7 verschiedene Stadien eingeteilt, die gemeinhin mit römischen Ziffern bezeichnet werden. Die Bildung von Proteinkristallen beginnt im Stadium III.

Bei Betrachtung der morphologischen Änderungen während der Sporulation wird deutlich, daß dabei zahlreiche Vorgänge nacheinander erfolgen. Wird die Expression eines an der Sporulation beteiligten Gens durch eine Mutation blockiert, dann werden oft die nachfolgenden Gene ebenfalls nicht mehr exprimiert. Bei vielen spo-Mutanten bricht deshalb die Sporulation in einem bestimmten Stadium ab. Diese Eigenschaft wird herangezogen, um die Mutation zu charackterisieren. Demzufolge ist eine spo III-Mutante im Stadium III der Sporulation blockiert, d.h. die Sporulation bleibt im Stadium III stehen. Bei einer spo VI-Mutante bleibt die Sporulation im Stadium VI stehen, und eine spo 0-Mutante ist nicht fähig vom vegetativen Wachstum zur Sporulation umzuschalten. Es gibt aber auch Ausnahmen. So ist z.B. die Expression der Gene, die an der Lyse des Sporangiums im Stadium VII verantwortlich sind, durch den Ausfall von spo III oder spo V-Genen nicht betroffen. Wie schon erwäht, hat die Synthese der Kristalle im Stadium III schon begonnen. Ein Defekt im einem Gen, das im Stadium III oder später benötigt wird, sollte deshalb die Synthese von Kristallen nicht beeinflussen, wohl aber die Entstehung einer intakten hitzeresistenten Spore.

Isolierung von spo-Mutanten

Kolonien von B.thuringiensis machen auf Nährager im Laufe einiger Tage beträchtliche Veränderungen durch. Beginnend im Zentrum trüben sich die zunächst bräunchlichen Kolonien wieß ein. Zuweilen, bei sehr großen Kolonien, fällt das Zentrum der Kolonie zusammen und es bildet sich eine Art Krater. Diese Veränderungen in der Koloniemorphologie werden durch die Sporulation bewirkt. Bei den Zellen im Zentrum einer Kolonie werden die Substrate zuerst knapp, sie beginnen deshalb als erste ihren Stoffwechsel auf Sporulation umzuschalten. Ein geübter, mit dem Organismus vertrauter Mikrobiologe kann deshalb leicht erkennen, ob eine Kolonie sporuliert. Bei B.thuringiensis ist dieses Stadium bei 28°C nach 3 bis 4 Tagen erreicht. Kolonien von Mutanten, die nicht mehr zur Sporulation befähigt sind, werden nicht trübe, sondern Z.T. etwas durchscheinender. Auf diese Weise ist es möglich nach einer Mutagenese z.B. mit NTG tausende von Einzelkolonien ohne übermäßigen Zeitaufwand durchzumustern.

Nicht alle Kolonien mit veränderter Morphologie sind spo-Mutanten. Deshalb werden zur weiteren Charakterisierung von jeder veränderten Kolonne Proben abgenommen und unter dem Phasenkontrastmikroskop durchgemustert. Dabei ist leicht zu erkennen, ob es sich um spo-Mutanten handelt und ob noch Proteinkristalle produziert werden. In einer Kolonie von B.thuringiensis erfolgen Wachstum und Sporulation asynchron, d.h. wenn im Zentrum einer Kolonie die Sporulation abgeschlossen ist, sind die Zellen an ihrem Rand noch im Zustand des vegetativen Wachstums. Deshalb ist es auch dann möglich eine Mutante weiter zu propagieren, wenn der genetische Defekt in der Sporulation die Zerstörung der reifenden Spore zur Folge hat.

Auf diese Weise wurden zahlreiche asporogene Mutanten von Stamm A-60 und von B.thuringiensis isoliert. Viele davon haben eine hohe Reversionsrate zurück zum sporogenen Ausgangsstamm. Für die weitere Entwicklung wurden nur die zwei Mutanten HA-1 und HA-5 bearbeitet, da bei ihnen keine Reversion zum sporogenen Ausgangsstamm beobachtet wurde.

Beschreibung der Eigenschaften von spo-Mutanten Stamm HA-1

Die erste asporogene Mutante vom Stamm A-60, welche eingehender bearbeitet wurde, wird als Stamm HA-1 bezeichnet. Eine sporulierte Kultur dieser Mutante unterscheidet sich von einer entsprechenden Kultur des Stammes A-60 dadurch, daß an Stelle der lichtbrechenden Endosporen größere ovale, von einem Exosporium umhüllte Zellen zu sehen sind. Beide Kulturen enthalten die für mückenpathogene Stämme typischen unregelmäßog geformten parasporalen Kristalle.

Das Auftreten von insektizider Wirkung und hitzestabilen bzw. -labilen Endosporen während Fermentationen von Stamm A-60 und der Mutante HA-1 läßt sich ebenfalls optisch verfolgen; es zeigt, daß die Mutante ebenso schnell wächst wie der Ausgangsstamm; auch die Bildung der Toxizität erfolgt synchron. Die Bildung von hitzeresistenten Sporen bleibt bei der Mutante jedoch aus.

Die elektronenmikroskopische Aufnahme zeigt, daß es sich bei den beschriebenen Zellen um defekte Sporen handelt, da sie alle für die Spore typischen Schichten der Sporenwand enthält. Die defekten Sporen unterscheiden sich von den intakten dadurch, daß das Cytoplasma nicht kondensiert ist, und der fehlenden Hitzeresistenz. Die Sporenrinde (Cortex) ist nicht wie bei Stamm A-60 eine ghleichmäßig dicke Schicht, welche die Spore gänzlich umhüllt, sondern sie scheint unterbrochen und die Spore nicht vollständig zu umhüllen.

Die Bildung der Sporenrinde erfolgt im Stadium V der Sporulation. Da bei der Mutante noch eine defekte Sporenrinde zu sehen ist, ohne daß eine Kondensation des Cytoplasmas zu beobachten ist, wird die Mutante als spo V-Mutante bezeichnet. Obwohl der Defekt in einem Gen des Stadiums V liegt, erfolgt die für das Stadium VII typische Lyse des Sporangiums. Die Expression der dafür benötigten Gene scheint nicht durch die spo V-Mutation beeinflußt zu werden.

Die hitzelabilen, defekten Sporen können am Ende einer Fermentation durch 10minütiges Erhitzen auf 80°C inaktiviert werden, ohne daß die Toxizität der Proteinkristalle abnimmt. Damit ist es möglich ohne Bestrahlung ein Rohprodukt zu bekommen, das der Gewässerschutzauflage genügt.

Der erfindungsgemäße Stamm HA-5

Die Mutante HA-1 hat den Nachteil, daß im Rohprodukt neben den Proteinkristallen noch defekte

4

Sporen auftreten, die eine beträchtliche Verdünnung des Wirkstoffes verursachen. Deshalb wurde nach weiteren Mutanten gesucht, die in einem früheren Stadium der Sporulation defekt sind. Während einer weiteren Durchmusterung von geeignet erscheinenden Kolonien wurde eine Mutante isoliert, die als HA-5 bezeichnet wurde. Wie man unter dem Mikroskop sieht, findet auch bei dieser Mutante eine Lyse des Sporangiums statt, wobei nur die typischen Proteinkristalle und keine Sporen freigesetzt werden. Zur genaueren Charakterisierung wurden bei einer Fermentation mit dieser Mutante zu verschiedenen Zeiten nach Ende des logarithmischen Wachstums Proben gezogen und Ultradünnschnitte der sporulierenden Zellen im Elektronenmikroskop untersucht. Die Präspore zeigt das am weitesten fortgeschrittene Stadium der Sporulation, das bei der Mutante HA-5 beobachtet werden konnte. Man erkennt die doppelte Membran und dazwischen die primäre Zellwand der Spore. Dies entspricht dem Entwicklungsstand, den die Spore im Stadium III hat. Diese Mutante wird deshalb als spo III-Mutante bezeichnet. Die primäre Zellwand der Spore hat dieselbe Struktur wie die Zellwand des Sporangiums. Es ist deshalb verständlich, daß Enzyme, die das Sporangium lysieren können, auch die Präspore abbauen, so daß am Ende einer Fermentation nur noch Proteinkristalle übrig bleiben.

Herstellung von ts-Mutanten
Unzulänglichkeiten der spo-Mutanten
Bei vielen Bacillus-Stämmen kann beobachtet werden, daß am Ende des logarithmischen Wachstums nicht alle Zelle sporulieren. Der Anteil an sporulierenden Zellen in einer Kultur, die sich in der stationären Phase befindet, kann auf weniger als 1% abfallen. Solche Stämme bezeichnet man als oligosporogen. Die Ursachen für dieses Phänomen können genetisch determiniert sein, sehr oft spielt auch die Zusammensetzung des Mediums eine Rolle. Auch bei Stamm A-60 sind in kulturen die sich im Stadium VII der Sporulation befinden, immer noch vegetativez Zellen zu sehen.
Bei der Fermentation der Mutante HA-5 tritt folgendes Phänomen auf. Am Ende der logarithmischen Wachstumsphase ist das Nährmedium erschöpft und die Zellen stellen, das Wachstum ein. Die überweigende Mehrzahl der Zellen stellt ihren Stoffwechsel auf Sporulation um, die restlichen Zellen ruhen. Am Ende der Sporulation lysieren die Sporangien und die Präsporen. Dabei werden als Substrate geeignete Zellbestandteile in das Medium freigesetzt, worauf die noch vorhandenen vegetativen Zellen wider zu wachsen beginnen. Durch die Zunhame der Zahl der vegetativen Zellen wird die Konzentration der Proteinkristalle im Rohprodukt verringert. Da die vegetativen Zellen sehr viel größer als Proteinkristalle sind, ist der Verdünnungseffekt schon bei einer geringen Zunahme der vegetativen Zellen stark ausgeprägt. Bei der Fermentation mit der Mutante HA-5 kann das sekundäre Wachstum durch Wahl des richtigen Zeitpunkts der Ernte eingeschränkt werden, erfordert aber gegen Ende der Fermentation, häufiges Probenziehen und sorgfältige Überwachung. Dennoch enthalten Rohpräparate, die mit der Mutante HA-5 hergestellt wurden immer noch vegetative Zellen.

Beschreibung des erfindungsgemäßen Stammes HA-9
B.thuringiensis wächst zwischen etwa 25 und 45°C. Es gibt eine Art von Mutanten, die als temperatursensitiv, abgekürzt ts-Mutanten bezeichnet werden. Solche Mutationen können in verschiedenen Genen auftreten. Sie bewirken, daß die Zelle z.B. bei 28°C ein intaktes und z.B. bei 43°C ein defektes Genprodukt synthetisiert.
Um das sekundäre Wachstum bei der asporogenen Mutante HA-5 am Ende der Sporulation zu verhindern, wurde diese nochmals mit NTG behandelt und unter den überlebenden Zellen auf solche selektioniert, die einen ts-Defekt im vegetativen Wachstum haben. Alle Mutanten, die isoliert wurden, wachsen bei unter 30, z.B. bei 28°C, jedoch nicht bei über 35, z.B. bei 43°C und besitzen noch die spo III-Mutation. Läßt man die Mutanten z.B. bei 28°C wachsen bis die Sporulation begonnen hat, und erhöht dann die Temperatur auf z.B. 43°C, wird die Synthese der Kristalle nicht beeinträchtigt. Unter den Mutanten, die diese Bedingungen erfüllen, befinden sich einige, die einen zusätzlichen Effekt aufweisen. Erhöht man die Temperatur auf 43°C, dann hört das Wachstum nicht nur auf, sondern die vegetativen Zellen beginnen außerdem zu lysieren, so daß ihre Zahl schnell abfällt.
Bei der Mutante HA-9 ist dieser Effekt besonders ausgeprägt. Wird bei dieser Mutante während der Sporulation die Temperatur auf 43°C erhöht, dann lysieren innerhalb weniger Stunden alle vegetativen Zellen in der Kultur. Die durch Zentrifugation des Kulturmediums gewonnene Biomasse enthält weder Sporen noch vegetative Zellen, sondern fast ausschließlich Proteinkristalle.

Beispiel
Beschreibung des Verfahrens zur Herstellung von mückentoxischen Proteinkristallen mit Hilfe der Mutante HA-9
Es wurde ein Fermentationsmedium entwickelt, das Sojamehl, Hefeautolysat, Kartoffelstärke und Mineralsalze enthält. Die Zusammensetzung des Mediums ist in der Tabelle wiedergegeben.
Zwei 500 ml-Kolben mit je einer Schikane, die jeweils 250 ml Fermentationsmedium enthalten, wurden mit der Mutante HA-9 angeimpft. Die Kolben wurden dann 16 h lang bei 28°C geschüttelt. Die zum Animpfen der Kolben verwendete Kolonie wurde auf Hefeextrakt-Glucose-Pepton-Agar bei 28°C angezogen und war nicht älter als 48 h.
Mit den Übernachtkulturen wurde ein auf 28°C temperierter 25 1-Fermenter mit Fermentationsmedium

5

angeimpft. Dabei wurden Fermenter mit Umwurf verwendet. Die Drehzahl des Rühwerks wurde auf 1200 Upm und die Belüftung auf 25 l/min einreguliert.

Nach 7 h befanden sich die Zellen im Vorfermenter in der mittleren bis späten logarithmischen Wachstumsphase und wurden zum Animpfen des Hauptfermenters benützt. Dazu wurde der Inhalt des Vorfermenters in den Hauptfermenter umgepumpt, der 200 l sterilisiertes und auf 28°C temperiertes Fermentationsmedium enthielt. Die Drehzahl der Turbine wurde auf 1200 Upm und die Belüftung auf 6 m³/h einreguliert. Da in der logarithmischen Wachstumsphase eine starke Ansäuerung des Mediums erfolgt, wurde der pH-Wert durch Zupumpen von 2 N NaOH bei pH 7.4 gehalten. Gegen Ende der logarithmischen Wachstumsphase steigt der pH-Wert in der Kultur auf ph > 8.0. Nachdem die Synthese der Proteinkristalle eingeleitet war, wurde die Temperatur im Fermenter nach 19 h auf 43°C erhöht. Nach insgesamt 26 h wurde die Fermentation abgebrochen und die Proteinkristalle durch Zentrifugation geerntet.

TABELLE

| | | |
|---|---|---|
| Sojamehl entfettet | 10,0 | g/l |
| Kartoffelstärke | 5,0 | g/l |
| Hefeautolysat | 2,0 | g/l |
| $K_2HPO_4$, wasserfrei | 1,0 | g/l |
| $MgSO_4 \times 7\ H_2O$ | 0,3 | g/l |
| $CaCl_2 \times 6\ H_2O$ | 0.08 | g/l |
| $MnCl_2 \times 4\ H_2O$ | 0,05 | g/l |
| CuCl | 0,005 | g/l |
| $ZnCl_2$ | 0,005 | g/l |
| $FeCl_3$ | 0,005 | g/l |

Zur Anwendung als Insektizid wird das erfindungsgemäß erhaltene insektizid wirkende Präparat bzw. Toxin in an sich bekannter Weise mit üblichen Additiven (Trägerstoffe, Haftmittel, Netzmittel etc.) versetzt und in eine geeignete Anwendungsform übergeführt. Das so formulierte Insektizid kann in Form eines Spritzpulvers, einer Suspension, als Granulat o.ä. eingesetzt werden.

Anwendungsbeispiel

Aedes aegypti, Gelbfiebermücke

Versuchsart:
Dauerkontakt/Fraß; Aedes aegypti

Versuchsdurchführung:
Kunstoffbecher mit 250 ml Inhalt. Durchmesser 8 cm, werden mit 200 ml Leitungswasser von ca. 23°C gefüllt und mit 20 Aedes-Larven im 2. Larvenstadium besetzt. Darauf gibt man die Prüfsubstanz als wäßrige Emulsion oder Suspension in das Gefäß und bestimmt nach 24 Stunden die Mortalität in den Gefäßen und errechnet den $LC_{50}$-Wert.

| | $LC_{50}$-Wert (ppm) nach 24 h | |
|---|---|---|
| | Rohware | Gefriergetrocknet |
| Wildtyp | 0.15 | 0.018 |
| HA5 | 0.030 | 0.0065 |
| HA9 | 0.032 | 0.009 |

# EP 0 278 035 B1

**Patentansprüche**

1. Sporenlose Mutante des Bacillus thuringiensis serovar.israelensis gemäß der unter der Nummer DFSM 3439 bei der Deutschen Sammlung von Mikroorganismen hinterlegten Probe.

2. Sporenlose Mutante des Bacillus thuringiensis serovar.israelensis gemäß der unter der Nummer DSM 3440 bei der Deutschen Sammlung von Mikroorganismen hinterlegten Probe.

3. Verfahren zur Herstellung ballastarmer und sporenfreier mückentoxischer Präparate aus Bacillus thuringiensis serovar.israelensis, dadurch gekennzeichnet, daß man, gegebenenfalls nach Einwirkung von die Mutationsrate erhöhenden Mitteln aujs wachsenden Bakterienkolonien diejenigen ausliest, die bei einer Fermentationstemperatur von unter 30°C thermisch instabile Sporen bilden, deren vegetative Zellen vermehrt und aus dem Substrat in an wich bekannter Weise das Toxin gewinnt.

4. Verfahren zur Herstellung konzentrierter, ballastarmer und sporenfreier mückentoxischer Proteinpräparate aus Bacillus thuringiensis serovar.israelensis, dadurch gekennzeichnet, daß man, gegebenenfalls nach Einwirkung von die Mutationsrate erhöhenden Mitteln, aus wachsenden Bakterienkolonien diejenigen ausliest, die ausschließlich Präsporen bilden, diese in einem geeigneten Kulturmedium bei einer Temperatur von unter 30°C vermehrt, bis die Sporulation eingetreten ist und danach bei einer Temperatur von über 35°C hält, die zur Autolyse der Sporen führt, sowie das Toxin aus dem Kulturmedium gewinnt.

5. Verwendung des nach dem Verfahren des Anspruchs 3 erhältlichen Toxins zur Bekämpfung von Stechmücken.

6. Verwendung des nach dem Verfahren des Anspruchs 4 erhältlichen Toxins zur Bekämpfung von Stechmücken.

7. Insektizid, dadurch gekennzeichnet, daß es als aktiven Wirkstoff Bacillus thuringiensis serovar.israelensis DSM 3439 oder ein hieraus erhältliches Toxin enthält.

8. Insektizid, dadurch gekennzeichnet, daß es als aktiven Wirkstoff Bacillus thuringiensis serovar.israelensis DSM 3440 oder ein hieraus erhältliches Toxin enthält.


**Revendications**

1. Mutants, libres de spores, du Bacillus thuringiensis serovar. israelensis selon l'essai déposé au recueil allemand des microorganismes sous le numéro DSM 3439.

2. Mutants, libres de spores, du Bacillus thuringiensis serovar. israelensis selon l'essai déposé au recueil allemand des microorganismes sous le numéro DSM 3440.

3. Procédé d'obtention de préparations de Bacillus thuringiensis serovar. israelensis toxiques aux moustiques, sans pores et pauvres en stériles, caractérisé par le fait qu'on sépare des colonies de batéries croissantes, éventuellement aprés action de moyens élevant le taux de mutation, celles qui, à une température de fermentation plus faible que 30 degrés C, forment des spores thermiquement instables dont les cellules végétatives se multiplicent et on extrait la toxine du substrat de manière connue en soi.

4. Procédé d'obtention de préparations protéiniques de Bacillus thuringiensis serovar. israelensis toxiques aux moustiques, sans spores, pauvres en stériles et concentrées, caractérisé par le fait qu'on sépare des colonies de batéries croissantes, éventuellement après action de moyens élevant le taux de mutation, celles qui forment exclusivement des préspores, qu'on multiplie ceuxci dans un milieu de culture approprié, à une température inférieure à 30 degrés C jusqu'à ce que la sporulation soit engagée, on maintient ensuite, à une température supérieure à 35 degrés C, qui conduit à l'autolyse des spores et extrait la toxine du milieu de culture.

5. Utilisation de la toxine obtenue par le procédé selon la revendication 3 pour lutter contre les moustiques.

6. Utilisation de la toxine obtenue par le procédé selon la revendication 4 pour lutter contre les moustiques.

7. Insecticide, caractérisé par le fait qu'il contient, comme principe actrif, du Bacillus thuringiensis serovar. israelensis DSM 3439 ou une toxine extraite de lui.

8. Insecticide, caractérisé par le fait qu'il contient, comme principe actif, du Bacillus thuringiensis serovar. israelensis DSM 3440 ou une toxine extraite de lui.


**Claims**

1. An asporous mutant of Bacillus thuringiensis serovar. israelensis represented by the sample deposited under number DSM 3439 at the German Collection of Microorganisms.

2. An asporous mutant of Bacillus thuringiensis serovar. israelensis represented by the sample deposited under DSM 3440 at the German Collection of Microorganisms.

3. A process for the preparation of low-ballast and spore-free preparations, which are toxic for gnats, from Bacillus thuringiensis serovar. israelensis, which comprises selection, when appropriate after exposure to agents increasing the mutation rate, from growing bacterial colonies of those which, when fermented at below 30°C, form spores which are unstable to heat, propagation of their vegetative cells, and isolation of the toxin from the substrate in a conventional manner.

4. A process for the preparation of concentrated, low-ballast and spore-free protein preparations, which are toxic for gnats from Bacillus thuringiensis serovar. israelensis, which comprises selection, where appropriate after exposure to agents increasing the mutation rate, from growing bacterial colonies of those which form exclusively prespores, propagation of the latter in a suitable culture medium at below 30°C until sporulation has occurred, and then maintenance at above 35°C, which results in autolysis of the spores, and isolation of the toxin from the culture medium.

5. The use of the toxin obtainable by the process as claimed in claim 3 for controlling mosquitos.

6. The use of the toxin obtainable by the process as claimed in claim 4, for controlling mosquitos.

7. An insecticide which contains as active ingredient Bacillus thuringiensis serovar. israelensis DSM 3439 or a toxin obtainable therefrom.

8. An insecticide which contains as active ingredient Bacillus thuringiensis serovar. israelensis DSM 3440 or a toxin obtainable therefrom.